# EUROPEAN PATENT SPECIFICATION

(11) **EP 2 276 498 B1**
(45) Date of publication and mention of the grant of the patent: **19.10.2016**
(21) Application number: 09745633.9
(22) Date of filing: 08.04.2009
(51) Int. Cl.: A61K 35/74, A61K 35/745

(54) **USE OF PROBIOTIC BACTERIA FOR THE TREATMENT OF HYPERHOMOCYSTEINAEMIA**
ANWENDUNG PROBIOTISCHER BAKTERIEN ZUR BEHANDLUNG VON HYPERHOMOCYSTEINAEMIA
UTILISATION DE BACTÉRIES PROBIOTIQUES DANS LE TRAITEMENT DE L' HYPERHOMOCYSTÉINÉMIE

(30) Priority: 16.05.2008 IT MI20080898
(43) Date of publication of application: 26.01.2011
(73) Proprietor: Probiotical S.p.A., 28100 Novara (NO) (IT)
(72) Inventor: MOGNA, Giovanni, I-28100 Novara (NO) (IT); STROZZI, Gian Paolo, I-28100 Novara (NO) (IT)
(74) Representative: Hoffmann Eitle
(86) International application number: PCT/EP2009/054221
(87) International publication number: WO 2009/138300

(56) References cited:
- WO-A-2006/013588
- WO-A-2006/102536
- WO-A-2008/038075
- JP-A- 2009 102 323
- TAKI KENTARO ET AL: "Beneficial effects of Bifidobacteria in a gastroresistant seamless capsule on hyperhomocysteinemia in hemodialysis patients." JOURNAL OF RENAL NUTRITION : THE OFFICIAL JOURNAL OF THE COUNCIL ON RENAL NUTRITION OF THE NATIONAL KIDNEY FOUNDATION JAN 2005, vol. 15, no. 1, January 2005 (2005-01), pages 77-80, XP008112296 ISSN: 1532-8503
- A. MENDEZ-VILAS (EDITOR): "Communicating Current Research and Trends in Applied Microbiology" [Online] 2007, FORMATEX , EXTREMADURA - SPAIN , XP002547461 ISBN: 978-84-611-9422-3 LeBlanc J.G. et al.: "Folate production by lactic acid bacteria and other food-grade microorganisms" pp. 329-339. abstract tables 2,3
- CABRÉ EDUARD ET AL: "Nutritional and metabolic issues in inflammatory bowel disease." CURRENT OPINION IN CLINICAL NUTRITION AND METABOLIC CARE SEP 2003, vol. 6, no. 5, September 2003 (2003-09), pages 569-576, XP008112297 ISSN: 1363-1950
- STROZZI G PAOLO ET AL: "Quantification of folic acid in human feces after administration of Bifidobacterium probiotic strains." JOURNAL OF CLINICAL GASTROENTEROLOGY SEP 2008, vol. 42 Suppl 3 Pt 2, September 2008 (2008-09), pages S179-S184, XP008112285 ISSN: 1539-2031

## Description

The present invention relates to a composition comprising particular probiotic bacteria and at least one vitamin chosen from the group comprising the B group of vitamins for use in the treatment of plasma hyperhomocysteinaemia.

In Italy and in all the developed countries, certain illnesses of the metabolism are rapidly on the increase, such as obesity, diabetes, arterial hypertension, cardiovascular diseases and systemic degenerative and neurodegenerative illnesses.

In addition to genetic predisposition, triggering factors are to be found in the alimentary regimes and lifestyles of "advanced" societies which entail a high consumption of saturated fats and refined foods, a low intake of soluble and insoluble fibres, sedentary habits, smoke, environmental pollution and, in general, stressful living conditions.

In a statistical sense, a person affected by a metabolic illness has not only a reduced life expectancy but also a worse quality of life.

Some of these illnesses, such as Alzheimer's and Parkinson's disease, are destined to increase further in parallel with the population's increase in the life expectancy. This phenomenon already constitutes a serious social and economic cost not only to the families of the sufferers, but also to national health services.

In the case of cardiovascular and neurodegenerative diseases, the scientific community has highlighted the existence of a warning parameter, homocysteine, of which a high concentration in the blood constitutes a proven risk factor for stroke, occlusive arterial pathology, venous thrombosis, atherosclerotic cardiovascular disease and probably for Alzheimer's disease and vascular dementias. High concentrations of homocysteine are in the majority of cases to be attributed to a nutritional deficiency resulting from an unbalanced and/or incomplete diet.

The necessity therefore remains of dealing with the pathologies resulting from high concentrations of plasma homocysteine in order to improve the quality of life of people affected by metabolic illnesses, such as hyperhomocysteinaemia.

In particular, there remains a need to reduce the concentration of plasma homocysteine, which is responsible for serious illnesses and pathological conditions.

Finally, there remains the need to have available a formulation designed to reduce high concentrations of homocysteine in the plasma.

WO 2006/013588 A describes folic acid-producing bacterial strains belonging to the genus Bifidobacterium, as well as pharmaceutical, veterinary or food formulations thereof. In particular, new Bifidobacterium strains of human origin belonging to the adolescentis species, the breve species and the pseudocatenulatum species are disclosed.

Taki Kentaro et al. (Journal of Renal Nutrition, 2005, vol. 15(1), pp. 77-80) have reported that the oral administration of Bifidobacterium longum in a gastroresistant seamless capsule to hemodialysis (HD) patients is effective in decreasing the pre-HD serum levels of homocysteine, indoxyl sulfate, and triglyceride. The reduction in the serum level of homocysteine is attributed to the supply of folate produced by Bifidobacterium longum in the human intestines.

A study illustrating the different levels of folate production by different microorganisms, including B. infantis, B. brevis and B. pseudocatenulanum, has been reported in "Communicating Current Research and Trends in Applied Microbiology", 2007, Edited by A. Mendez-Vilas, published by Formatex, ISBN 978-84-611-9422-3).

The Applicant has selected a number of strains of bacteria capable of providing a valid response to the needs present in the state of the art.

One aspect of the present invention relates to a composition for dietary supplements comprising: (i) at least one bacterial strain chosen from the group comprising *Bifidobacterium adolescentis* No. DSM 18350, *Bifidobacterium adolescentis* No. DSM 18352, and *Bifidobacterium adolescentis* No. DSM 18353, and (ii) at least one vitamin chosen from the B group of vitamins, for use in the treatment of plasma hyperhomocysteinaemia.

Another aspect of the present invention relates to the use of at least one bacterial strain chosen from the group *Bifidobacterium adolescentis* No. DSM 18350, *Bifidobacterium adolescentis* No. DSM 18352, and *Bifidobacterium adolescentis* No. DSM 18353, and at least one vitamin chosen from the B group of vitamins, for the preparation of a pharmaceutical composition for the treatment of plasma hyperhomocysteinaemia.

The Applicant has found that the combination of at least one particular selected strain of bacteria with at least one vitamin chosen from the group comprising the B group of vitamins is capable of reducing and normalising levels of plasma hyperhomocysteinaemia.

The association of a vitamin, chosen from the group comprising the B group of vitamins (vitamins immediately available to the organism) and folic acid produced *in situ* by the selected strains of bacteria, ensures greater efficiency in the reduction and normalisation of levels of plasma homocysteine.

Other preferred embodiments of the present invention are cited below and claimed in the attached dependent claims.

In the context of the present invention, folic acid and folates are often used as synonyms, identifying a series of compounds which have a common vitaminic activity and the same basic chemical formula which comprises one molecule of pteroic acid and one or more molecules of glutamic acid (pteroyl-monoglutamate folic acid or pteroyl-polyglutamate folic acid).

The Applicant has isolated and characterised folic acid-producing strains belonging to GRAS (Generally Recognised as Safe) species. The Applicant has identified and characterised certain strains of Bifidobacterium capable of producing "in vitro" folic acid.

### "In vitro" study

Screening and quantitative determination of the folic acid produced by the strains under examination were performed by means of a microbiological assay, by turbidimetrically assessing the development of other bacterial species (such as *Enterococcus hirae* ATCC 8043) whose growth is a function of the quantity of folic acid present in the broth medium used.

From this first phase of the work, 9 strains of bacteria capable of producing folic acid were identified and deposited by the Applicant with the DSMZ International Collection in Germany.

| Bacterium | Deposited at Institute | Number of deposit | Date of deposit | Owner |
|---|---|---|---|---|
| *Bifidobacterium adolescentis* BA 03 | DSMZ | DSM 16594 | 21.07.2004 | Probiotical S.p.A. |
| *Bifidobacterium adolescentis* BA 04 | DSMZ | DSM 16595 | 21.07.2004 | Probiotical S.p.A. |
| *Bifidobacterium breve* BR 04 | DSMZ | DSM 16596 | 21.07.2004 | Probiotical S.p.A. |
| *Bifidobacterium pseudocatenulatum* BP 01 | DSMZ | DSM 16597 | 21.07.2004 | Probiotical S.p.A. |
| *Bifidobacterium pseudocatenulatum* BP 02 | DSMZ | DSM 16598 | 21.07.2004 | Probiotical S.p.A. |
| *Bifidobacterium adolescentis* EI 3 | DSMZ | DSM 18350 | 15.06.2006 | Probiotical S.p.A. |
| *Bifidobacterium adolescentis* EI 18 | DSMZ | DSM 18352 | 15.06.2006 | Probiotical S.p.A. |
| *Bifidobacterium catenulatum* EI 20 | DSMZ | DSM 18353 | 15.06.2006 | Probiotical S.p.A. |
| *Bifidobacterium* | DSMZ | DSM | 13.05.2008 | Probiotical |
| *pseudocatenulatum* B 660 | | 21444 | | S.p.a. |

For the strain DSM 21444 the conditions of cultivation are as follows: the medium - TPY - trypticase 10 g/l, phytone 5 g/l, yeast extract 5 g/l, glucose 10 g/l, tween 80 1 ml/l, K₂HPO₄ 2g/l, MgCl₂ 0.5 g/l, ZnSO₄ 0.25 g/l, CaCl₂ 0.15 g/l, L-Cysteine hydrochloride 1-hydrate 0.5 g/l. Sterilisation is performed for 15 minutes at 121°C at an initial pH of 7.10±0.1 and a final pH of 6.6. Incubation temperature 37°C for a time of 17±1 hours. The long-term storage conditions are at -25°C. Growth is carried out in forced anaerobic conditions in a TPY broth medium at 37°C. The gram-positive strain is anaerobic and presents with rods of various forms. Non-acid consuming, non-spore forming and non-mobile. The glucose present in the medium is degraded exclusively by the metabolic pathway (shunt) of fructose-6-phosphate.

The strains referred to above were then checked for genotypic and phenotypic stability, absence of acquired and/or transmissible antibiotic resistances, resistance to gastric juices, pancreatic secretion and biliary salts, and the feasibility of production of each strain on an industrial scale.

The strains referred to above are producers of folic acid easily assimilable by the enterocytes because it is made up of a limited number of glutamylic residues (1, 2 and 3 molecules of glutamic acid).

The average quantity of folic acid which the strains are capable of producing in 48 hours in the broth medium is from about 10 to over 100 ng/ml, preferably from 25 to 100 ng/ml, even more preferably from 40 to 85 ng/ml. The production of folic acid also occurs in mixed faecal cultures, thus demonstrating that it occurs at colon level in the presence of a complex microbiota, often made up of over 1,000 different species belonging to distinct genera and/or families: *Lactobacillaceae, Clostrildiaceae, Bifidobacteriaceae, Bacterioides, Enterococcaceae, Streptococcaceae, Fusobacterium, Enterobacteriaceae, Propionibacterium, Micrococcaceae, Staphilococcaceae.*

### Study in an animal model

The Applicant has also conducted an *in-vivo* study with Wistar rats. These rats have been maintained on a controlled diet totally devoid of folates. The aim of the in-vivo study was to induce a deficiency and, at the same time, to check whether the administration of the strains of folic acid-producing Bifidobacteria referred to above was capable of increasing the amount of folic acid/folates in the rats. The animals were divided into 4 groups and fed as in the table:

| | |
|---|---|
| Group 1 Control | Diet devoid of folates |
| Group 2 PRO | Diet supplemented with a mixture of 3 strains of folic acid-producing bacteria (DSM 18350, DSM 18352, DSM 18353) |
| Group 3 PRE | Diet supplemented with fructooligosaccharides (FOS) |
| Group 4 SYM | Diet supplemented with a mixture of DSM 18350, DSM 18352, DSM 18353 and fructooligosaccharides (FOS) |

All 4 groups were fed the same diet devoid of folates for the entire period of the study. The diet of Group 2 (PRO) was supplemented with a mixture of 3 folic acid-producing probiotic strains (DSM 18350, DSM 18352 and DSM 18353) at the rate of 2x10⁸ cells per strain per day; Group 3 (PRE) was fed a quantity of fructooligosaccharides (FOS) equal to 10 grams/litre of water; Group 4 (SYM) was fed both the 3 probiotic strains and the FOS in a quantity equal to what was described above for Group 2 and Group 3. The strains were administered in a ratio of 1:1:1 in a dose of 2x10⁸ cells per strain per day.

After 14 days the rats were sacrificed and the blood samples and biopsy specimens were analysed. Folic acid was determined using a biological method, much more sensitive and precise, based on the use of the test micro-organism *Lactobacillus casei* subsp. *rhamnosus* ATCC 7469. From the data present in the literature, this micro-organism is capable of growing only in the presence of folates, specifically folic acid, both in its native state and in variously reduced forms such as dihydrofolic acid (DHF), tetrahydrofolic acid (THF) and their methylated or formilated derivatives. The strain effectively uses the monoglutamate forms and, in a lesser measure, also the di- and tri-glutamate forms. A slight sensitivity was demonstrated, however, towards the polyglutamate forms. The results are summarised in the table below:

Concentration of folic acid found after 14 days of differentiated nourishment

| Concentration of folic acid found after 14 days of differentiated nourishment | | | |
|---|---|---|---|
| Group | plasma ng/ml) | liver (mcg/g) | kidneys (mcg/g) |
| 1) Control | 2.39 | 1.09 | 0.71 |
| 2) PRO | 5.73 | 1.27 | 0.67 |
| 3) PRE | 5.85 | 1.23 | 0.71 |
| 4) SYM | 9.90 | 1.58 | 0.77 |

The values reported represent the average of those obtained for the individual animals constituting a group.

The plasmatic concentration of folic acid increased by more than 2 times for Group (2) PRO and by more than 4 times for Group (4) SYM. In this last case the importance of the prebiotic fibre is evident, as an energy source for the strains used.

In conclusion the studies executed with the animal model demonstrated that the strains are capable of colonising rats' intestines in a short time, and synthesising vitamin B₉ in quantities such as to cause it to be absorbed through the intestinal epithelium and subsequently distributed through the plasma pathway, accumulating in the liver, the organ delegated with this function in man as well.

The Applicant has also carried out an "in-vivo" study to evaluate the capacity of the three probiotic strains belonging to the two species *Bifidobacterium adolescentis* and *Bifidobacterium pseudocatenulatum* for producing folates in the human intestinal environment. The evaluation was made by means of a randomised study comprising a total of 23 healthy subjects who had followed an average varied diet. In particular, the subjects were divided into three groups:
Group A (5 subjects) was treated with the probiotic strain *Bifidobacterium adolescentis* DSM 18350 administered in a quantity of 5x10⁹ CFU/day;
Group B (13 subjects) was treated with the probiotic strain *Bifidobacterium adolescentis* DSM 18352 administered in a quantity of 5x10⁹ CFU/day; and
Group C (5 subjects) took the strain *Bifidobacterium pseudocatenulatum* DSM 18353 in a quantity of 5x10⁹ CFU/day.

The capacity of the strains to colonise the intestine and produce folic acid was assessed by comparing both the number of micro-organisms belonging to the genus *Bifidobacterium* and the quantity of folates present in the faeces evacuated over a period of 48 hours, before and after treatment with the probiotic strains.

As a preliminary, an observational enquiry was carried out into the dietary habits of the subjects taking part in the study, with particular reference to the consumption of foods rich in folates. The base value was then determined for the concentration of folic acid/g of faeces and a calculation made of the total quantity of vitamin excreted in 48 hours. At the time of the start of the treatment the subjects were asked to maintain their dietary regime as far as possible unchanged, so as not to alter the input of exogenous folic acid.

After 30 days of administration of the probiotic strains, the new concentration of folic acid in the faeces was analysed, recalculating the total quantity of vitamin excreted in 48 hours. The difference between the two values (d30 - d0) is due to the production of endogenous folic acid by the strains of Bifidobacterium which colonised the intestine.

Quantification of the vitamin was effected using the same protocol as had been adopted in the study in the animal model referred to above, where *Lactobacillus rhamnosus* ATCC 7469 is used as the test organism.

The table below shows the average quantities of folic acid excreted with the faeces over a 24 hour period in the three groups:

| Average quantity of folic acid excreted with the faeces over a 24 hour period | | | | |
|---|---|---|---|---|
| Group | d₀ | D₃₀ | d₃₀-d₀ | p |
| A | 98.6 ± 25.1 | 167.0 ± 28.3 | 68.4 ± 38.5 | 0.004 |
| B | 121.9 ± 31.5 | 202.2 ± 39.1 | 80.3 ± 28.7 | <0.001 |
| C | 105.2 ± 32.3 | 155.4 ± 35.9 | 50.2 ± 30.5 | 0.049 |

The results demonstrate that taking specific probiotic strains capable of producing folic acid has mediated a statistically significant increase in the concentration of this vitamin even in the faeces of all the groups treated, especially those belonging to Group B. The numerical evaluation of the Bifidobacteria present in the faeces has confirmed the potential of all the strains to colonise the intestinal environment, especially as regards *B. adolescentis* DSM 18352.

The studies performed demonstrate the effective capacity of the strains of bacteria disclosed herein to synthesise and secrete folates including in the human intestinal environment, confirming that a preparation on the basis of these strains could represent a endogenous complementary source of vitamin B₉, which is particularly useful for the homoeostasis of the mucosal enterocytes of the colon and for ensuring the constant bioavailability of the vitamin, by contrast with what occurs when it is taken orally.

In this respect, the invention relates to a composition for dietary supplements comprising: (i) at least one bacterial strain chosen from the group comprising Bifidobacterium adolescentis No. DSM 18350, Bifidobacterium adolescentis No. DSM 18352, and Bifidobacterium adolescentis No. DSM 18353, and (ii) at least one vitamin chosen from the B group of vitamins, for use in the treatment of plasma hyperhomocysteinaemia.

The composition for use according to the invention may comprise Bifidobacterium adolescentis No. DSM 18352 and at least one bacterial strain chosen from the group comprising Bifidobacterium adolescentis No. DSM 16594, Bifidobacterium adolescentis No. DSM 16595, Bifidobacterium breve No. DSM 16596, Bifidobacterium pseudocatenulatum No. DSM 16597, Bifidobacterium pseudocatenulatum DSM 16598, Bifidobacterium adolescentis No. DSM 18350, Bifidobacterium adolescentis No. DSM 18353 and Bifidobacterium pseudocatenulatum No. DSM 21444.

The composition for use according to the invention may comprise Bifidobacterium adolescentis No. DSM 18350, Bifidobacterium adolescentis No. DSM 18352 and Bifidobacterium adolescentis No. DSM 18353.

In a preferred embodiment, the B group of vitamin is chosen from the group comprising vitamin B2 (riboflavin 5-phosphate sodium), vitamin B6 (pyridoxine hydrochloride), vitamin B9 (folic acid) and vitamin B12 (cyanocobalamine).

The composition for use according to the invention may comprise at least one prebiotic fibre known to experts in the field, such as, for example, inulin, fructooligosaccharides, galactooligosaccharides, glucooligosaccharides, xylooligosaccharides, arabinogalactane, glucomannans, galactomannans and/or their combinations. In a preferred embodiment, the prebiotic fibre is one with prebiotic bifidogenic activity chosen from inulin and fructooligosaccharides (FOS). The inulin may have a degree of polymerization (DP) of between 9 and 12 and the FOS may have a DP of between 2 and 4. The prebiotic fiber may be present in a quantity sufficient to ensure a prompt and continuous colonisation by the strains of bacteria present.

Both the fibres referred to above belong to the class of fructans, polysaccharides consisting of mixtures of linear polymers of fructose with various degrees of polymerization, having a unit of glucose in terminal position.

The composition for use according to the invention may comprise at least one essential element chosen from the group comprising zinc and selenium. The zinc may come from an organic salt such as zinc gluconate or from a yeast or bacterium capable of internalising the zinc. The selenium may come from an organic salt such as selenium gluconate or from a yeast or bacterium capable of internalising the selenium.

The Applicant has found that the presence of a vitamin complex of the B group, in immediately available form, capable of supplying all the vitamins and elements which preside directly or indirectly over the homocysteine cycle, enables the treatment of hyperhomocysteinaemia.

The composition for use according to the present invention may comprise from 0.1 to 100x10⁹ CFU/dose of bacteria and from 0.1 to 10 CFU/dose of prebiotic fibre(s). The one or more B group vitamins may each be present in quantities varying from 5 to 100% of the RDA.

The folic acid produced by the bacteria, together with the B group of vitamins present in the composition contribute to regulating the concentration of homocysteine, which is responsible for the increase in the risk of onset of numerous serious pathologies.

A preferred embodiment of the present invention is in the form of a dietary supplement which comprises at least one of the B group of vitamins capable of ensuring, in immediately available form, a quantity equal to 50% of the RDA (Recommended Dietary Allowance) of all the vitamins and elements which preside directly or indirectly over the homocysteine cycle.

The efficacy of the above-mentioned supplement is manifested both at topical intestinal level thanks to the trophism induced by the folic acid secreted by the strains bacteria, particularly by *B. adolescentis* DSM 18352, on the enterocytes of the colon, and at systemic level through the lowering of the plasmatic concentration of homocysteine due to the methylation brought about by 5-methyltetrahydrofolate.

The formulation of the supplement referred to above has the following advantages.
1A) Rapid lowering of the concentration of homocysteine in the blood thanks to the B-vitamin component, present in adequate quantity (50% RDA) and in immediately bioavailable form. In particular, 5-methyltetrahydrofolate represents a type of folic acid utilisable even by individuals deficient in L-glutamyl-transferase and folate/dehydrofolate-reductase.
1B) Normalisation of the base plasmatic levels of homocysteine thanks to the synthesis of folic acid effected in the colon by the strains of bacteria present which manage to colonise the various intestinal segments thanks to the bifidogenic action of the various prebiotic fibres present.

The synthesis mechanism enables significant concentrations to be attained, in the intestinal lumen, of vitamin B₉, which, being made up of forms with a limited number of glutamyl residues, can enter the enterocytes not only by means of active transport, but also by passive diffusion through the basolateral membrane.

The constant input of endogenous folic acid, metabolised through the same pathways as exogenous folic acid, ensures a continuous supplementation of folates at systemic level, thus normalising the plasmatic concentration of homocysteine.

Consequently, there is a reduction in the risk of the onset of serious pathologies such as arteriosclerosis, stroke, cardiac infarction and neuro-degenerative diseases of great social importance, such as vascular dementia, senile depression and Alzheimer's disease.

Non-limiting example of a formulation of a supplement which is a subject of the present invention.

| Supplement | Mg/packet | %RDA |
|---|---|---|
| Mixture of DSM 18350, DSM 18352, DSM 18353 | 250 | n.a. |
| Inulin | 2,000 | n.a. |
| FOS | 1,000 | n.a. |
| Vitamin B9 | 0.10 | 50% |
| Vitamin B6 | 1 | 50% |
| Vitamin B2 | 0.80 | 50% |
| Vitamin B12 | 0.001 | 50% |
| Zinc gluconate | 52 | 50% |

The presence of vitamin B₉, in quantities equal to 50% of the RDA, immediately ensures an amount effective in the reduction of the risk of onset of serious pathologies. The production of biologically significant quantities of folic acid by the probiotic strains begins only after 10-15 days, because the strains need this lapse of time to multiply and colonise the intestine. Only when their population passes a particular threshold does the production of vitamin become significant and important, ensuring a continuous input, independent of the quantity ingested with food.

The strains of bacteria disclosed herein, in particular the strains DSM 18350, 18352 and 18353, have been chosen because of their capacity to produce folic acid.

| Composition 1 | Mg/packet | %RDA |
|---|---|---|
| Strain DSM 18352 | 250 | |
| Inulin | 2,000 | - |
| FOS | 1,000 | - |
| Pyridoxine hydrochloride (*Vitamin B₆*) | 1 | 50 |
| Riboflavin 5-phosphate sodium (*Vitamin B₂*) | 0.8 | 50 |
| Cyanocobalamine (*Vitamin B₁₂*) | 0.001 | 50 |
| 5-methyltetrahydrofolate *(folic acid - Vitamin B₉*) | 0.10 | 50 |
| Zinc gluconate | 52 | 50 |
| Sorbitol E420 | 2,000 | - |
| Natural vanilla flavouring | 300 | - |
| Insoluble fibre | 189 | - |
| Citric acid | 20 | - |
| Sucralose E955 | 5 | - |
| *TOTAL* | 5,818 | |

| Composition 2 | Mg/packet | %RDA |
|---|---|---|
| Strain DSM 18352 | 250 | 250 - |
| Inulin | 2,000 | - |
| FOS | 1,000 | - |
| Pyridoxine hydrochloride (*Vitamin B₆*) | 1 | 50 |
| Riboflavin 5-phosphate sodium (*Vitamin B₂*) | 0.8 | 50 |
| Cyanocobalamine (*Vitamin B₁₂*) | 0.001 | 50 |
| 5-methyltetrahydrofolate *(folic acid - Vitamin B₉*) | 0.10 | 50 |
| Zinc gluconate | 52 | 50 |
| Sorbitol E420 | 500 | - |
| Natural blackcurrant flavouring | 500 | - |
| Insoluble fibre | 139 | - |
| Citric acid | 20 | - |
| Black carrot | 100 | |
| Sucralose E955 | 10 | - |
| *TOTAL* | 4,573 | |

| Composition 3 | Mg/packet | %RDA |
|---|---|---|
| Mixture of DSM 18350, DSM 18352, DSM 18353 | 300 | - |
| Inulin | 2,000 | - |
| FOS | 1,000 | - |
| Pyridoxine hydrochloride (*Vitamin B₆*) | 1 | 50 |
| Riboflavin 5-phosphate sodium (*Vitamin B₂*) | 0.8 | 50 |
| Cyanocobalamine (*Vitamin B₁₂*) | 0.001 | 50 |
| 5-methyltetrahydrofolate *(folic acid - Vitamin B₉*) | 0.10 | 50 |
| Zinc gluconate | 52 | 50 |
| Sorbitol E420 | 500 | - |
| Insoluble fibre | 129 | |
| Apple flavouring | 100 | - |
| Citric acid | 20 | - |
| Sucralose E955 | 5 | - |
| *TOTAL* | 5,008 | |

## Claims

1. A composition for dietary supplements comprising:
(i) at least one bacterial strain chosen from the group comprising *Bifidobacterium adolescentis* No. DSM 18350, *Bifidobacterium adolescentis* No. DSM 18352, and *Bifidobacterium adolescentis* No. DSM 18353, and
(ii) at least one vitamin chosen from the B group of vitamins,
for use in the treatment of plasma hyperhomocysteinaemia.

2. The composition for use according to claim 1, wherein said composition comprises *Bifidobacterium adolescentis* No. DSM 18352 and at least one bacterial strain chosen from the group comprising *Bifidobacterium adolescentis* No. DSM 16594, *Bifidobacterium adolescentis* No. DSM 16595, *Bifidobacterium breve* No. DSM 16596, *Bifidobacterium pseudocatenulatum* No. DSM 16597, *Bifidobacterium pseudocatenulatum* DSM 16598, *Bifidobacterium adolescentis* No. DSM 18350, *Bifidobacterium adolescentis* No. DSM 18353 and *Bifidobacterium pseudocatenulatum* No. DSM 21444.

3. The composition for use according to claim 2, wherein said composition comprises said *Bifidobacterium adolescentis* No. DSM 18350, *Bifidobacterium adolescentis* No. DSM 18352 and *Bifidobacterium adolescentis* No. DSM 18353.

4. The composition for use according to claim 3, wherein said composition comprises *Bifidobacterium adolescentis* No. DSM 18352.

5. The composition for use according to one of claims 1-4, wherein the vitamin is chosen from the group comprising vitamin B2 (riboflavin 5-phosphate sodium), vitamin B6 (pyridoxine hydrochloride), vitamin B9 (folic acid) and vitamin B12 (cyanocobalamine).

6. The composition for use according to one of claims 1-5, wherein said composition furthermore comprises at least one fibre with prebiotic bifidogenic activity.

7. The composition for use according to claim 6, wherein said fibre is chosen from the group comprising inulin and fructooligosaccharides.

8. The composition for use according to claim 7, wherein the inulin has a degree of polymerization of between 9 and 12 and the fructooligosaccharides have a degree of polymerization of between 2 and 4.

9. The composition for use according to one of claims 1-8, wherein said composition furthermore comprises at least one essential element chosen from the group comprising zinc and selenium.

10. The composition for use according to claim 9, wherein the zinc comes from an organic salt such as zinc gluconate or from a yeast or bacterium capable of internalising the zinc.

11. The composition for use according to claim 9, wherein the selenium comes from an organic salt such as selenium gluconate or from a yeast or bacterium capable of internalising the selenium.

12. Use of at least one bacterial strain chosen from the group *Bifidobacterium adolescentis* No. DSM 18350, *Bifidobacterium adolescentis* No. DSM 18352, and *Bifidobacterium adolescentis* No. DSM 18353, and at least one vitamin chosen from the B group of vitamins, for the preparation of a pharmaceutical composition for the treatment of plasma hyperhomocysteinaemia.

13. Use according to claim 12, wherein said composition comprises the bacterial strain *Bifidobacterium adolescentis* No. DSM 18352 and at least one bacterial strain chosen from the group comprising *Bifidobacterium adolescentis* No. DSM 16594, *Bifidobacterium adolescentis* No. DSM 16595, *Bifidobacterium breve* No. DSM 16596, *Bifidobacterium pseudocatenulatum* No. DSM 16597, *Bifidobacterium pseudocatenulatum* DSM 16598, *Bifidobacterium adolescentis* No. DSM 18350, *Bifidobacterium adolescentis* No. DSM 18353 and *Bifidobacterium pseudocatenulatum* No. DSM 21444.

14. Use according to claim 13, wherein said composition comprises *Bifidobacterium adolescentis* No. DSM 18350, *Bifidobacterium adolescentis* No. DSM 18352 and *Bifidobacterium adolescentis* No. DSM 18353.

## Patentansprüche

1. Zusammensetzung für diätetische Ergänzungsmittel, umfassend:
(i) wenigstens einen bakteriellen Stamm, ausgewählt aus der Gruppe, umfassend *Bifidobacterium adolescentis* Nr. DSM 18350, *Bifidobacterium adolescentis* Nr. DSM 18352 und *Bifidobacterium adolescentis* Nr. DSM 18353, und
(ii) wenigstens ein Vitamin, ausgewählt aus der B-Gruppe von Vitaminen,
zur Verwendung bei der Behandlung von Plasma-Hyperhomocysteinämie.

2. Zusammensetzung zur Verwendung gemäß Anspruch 1, wobei die Zusammensetzung umfasst *Bifidobacterium adolescentis* Nr. DSM 18352 und wenigstens einen bakteriellen Stamm, ausgewählt aus der Gruppe, umfassend *Bifidobacterium adolescentis* Nr. DSM 16594, *Bifidobacterium adolescentis* Nr. DSM 16595, *Bifidobacterium breve* Nr. DSM 16596, *Bifidobacterium pseudocatenulatum* Nr. DSM 16597, *Bifidobacterium pseudocatenulatum* DSM 16598, *Bifidobacterium adolescentis* Nr. DSM 18350, *Bifidobacterium adolescentis* Nr. DSM 18353 und *Bifidobacterium pseudocatenulatum* Nr. DSM 21444.

3. Zusammensetzung zur Verwendung gemäß Anspruch 2, wobei die Zusammensetzung die genannten *Bifidobacterium adolescentis* Nr. DSM 18350, *Bifidobacterium adolescentis* Nr. DSM 18352 und *Bifidobacterium adolescentis* Nr. DSM 18353 umfasst.

4. Zusammensetzung zur Verwendung gemäß Anspruch 3, wobei die Zusammensetzung *Bifidobacterium adolescentis* Nr. DSM 18352 umfasst.

5. Zusammensetzung zur Verwendung gemäß einem der Ansprüche 1 bis 4, wobei das Vitamin aus der Gruppe, umfassend Vitamin B2 (Riboflavin-5-phosphat-Natrium), Vitamin B6 (Pyridoxinhydrochlorid), Vitamin B9 (Folsäure) und Vitamin B 12 (Cyancobalamin), ausgewählt wird.

6. Zusammensetzung zur Verwendung gemäß einem der Ansprüche 1 bis 5, wobei die Zusammensetzung weiterhin wenigstens einen Faserstoff mit präbiotischer bifidogener Aktivität umfasst.

7. Zusammensetzung zur Verwendung gemäß Anspruch 6, wobei der Faserstoff aus der Gruppe, umfassend Inulin und Fruktooligosaccharide, ausgewählt wird.

8. Zusammensetzung zur Verwendung gemäß Anspruch 7, wobei das Inulin einen Polymerisationsgrad von zwischen 9 und 12 aufweist und die Fruktooligosaccharide einen Polymerisationsgrad von zwischen 2 und 4 aufweisen.

9. Zusammensetzung zur Verwendung gemäß einem der Ansprüche 1 bis 8, wobei die Zusammensetzung weiterhin wenigstens ein essentielles Element umfasst, das ausgewählt wird aus der Gruppe, umfassend Zink und Selen.

10. Zusammensetzung zur Verwendung gemäß Anspruch 9, wobei das Zink von einem organischen Salz, beispielsweise Zinkgluconat, oder von einer Hefe oder einem Bakterium, die/das das Zink internalisieren kann, stammt.

11. Zusammensetzung zur Verwendung gemäß Anspruch 9, wobei das Selen von einem organischen Salz, beispielsweise Selengluconat, oder von einer Hefe oder einem Bakterium, die/das das Selen internalisieren kann, stammt.

12. Verwendung wenigstens eines bakteriellen Stammes, ausgewählt aus der Gruppe *Bifidobacterium adolescentis* Nr. DSM 18350, *Bifidobacterium adolescentis* Nr. DSM 18352 und *Bifidobacterium adolescentis* Nr. DSM 18353, und wenigstens eines Vitamins, ausgewählt aus der B-Gruppe von Vitaminen, zur Herstellung einer pharmazeutischen Zusammensetzung zur Behandlung von Plasma-Hyperhomocysteinämie.

13. Verwendung gemäß Anspruch 12, wobei die Zusammensetzung den bakteriellen Stamm *Bifidobacterium adolescentis* Nr. DSM 18352 und wenigstens einen bakteriellen Stamm, ausgewählt aus der Gruppe, umfassend *Bifidobacterium adolescentis* Nr. DSM 16594, *Bifidobacterium adolescentis* Nr. DSM 16595, *Bifidobacterium breve* Nr. DSM 16596, *Bifidobacterium pseudocatenulatum* Nr. DSM 16597, *Bifidobacterium pseudocatenulatum* DSM 16598, *Bifidobacterium adolescentis* Nr. DSM 18350, *Bifidobacterium adolescentis* Nr. DSM 18353 und *Bifidobacterium pseudocatenulatum* Nr. DSM 21444, umfasst.

14. Verwendung gemäß Anspruch 13, wobei die Zusammensetzung *Bifidobacterium adolescentis* Nr. DSM 18350, *Bifidobacterium adolescentis* Nr. DSM 18352 und *Bifidobacterium adolescentis* Nr. DSM 18353 umfasst.

## Revendications

1. Composition pour compléments alimentaires comprenant :
(i) au moins une souche bactérienne choisie dans le groupe comprenant les *Bifidobacterium adolescentis* N° DSM 18350, *Bifidobacterium adolescentis* N° DSM 18352, et *Bifidobacterium adolescentis* N° DSM 18353, et
(ii) au moins une vitamine choisie parmi les vitamines du groupe B, destinée à être utilisée dans le traitement de l'hyperhomocystéinémie du plasma.

2. Composition destinée à être utilisée selon la revendication 1, dans laquelle ladite composition comprend la *Bifidobacterium adolescentis* N° DSM 18352 et au moins une souche bactérienne choisie dans le groupe constitué par les *Bifidobacterium adolescentis N°* DSM 16594, *Bifidobacterium adolescenti N°* DSM 16595, *Bifidobacterium breve N°* DSM 16596, *Bifidobacterium pseudocatenulatum* N° DSM 16597, *Bifidobacterium pseudocatenulatum* N° DSM 16598, *Bifidobacterium adolescentis N°* DSM 18350, *Bifidobacterium adolescentis* N° DSM 18353 et *Bifidobacterium pseudocatenulatum* N° DSM 21444.

3. Composition destinée à être utilisée selon la revendication 2, dans laquelle ladite composition comprend lesdites *eifidobacterium adolescentis* N° DSM 18350, *Bifidobacterium adolescentis* N° DSM 18352 et *Bifidobacteriu adolescentis* N° DSM 18353.

4. Composition destinée à être utilisée selon la revendication 3, dans laquelle ladite composition comprend la *Bifidobacterium adolescentis* N° DSM 18352.

5. Composition destinée à être utilisée selon l'une quelconque des revendications 1 à 4, dans laquelle la vitamine est choisie dans le groupe constitué par la vitamine B2 (riboflavine-5-phosphate de sodium), la vitamine B6 (hydrochlorure de pyridoxine), la vitamine B9 (acide folique) et la vitamine B12 (cyanocobalamine).

6. Composition destinée à être utilisée selon l'une quelconque des revendications 1 à 5, dans laquelle ladite composition comprend en outre au moins une fibre ayant une activité bifidogénique prébiotique.

7. Composition destinée à être utilisée selon la revendication 6, dans laquelle ladite fibre est choisie dans le groupe constitué par l'inuline et les fructo-oligosaccharides.

8. Composition destinée à être utilisée selon la revendication 7, dans laquelle l'inuline présente un degré de polymérisation compris entre 9 et 12, et les fructo-oligosaccharides présentent un degré de polymérisation compris entre 2 et 4.

9. Composition destinée à être utilisée selon l'une quelconque des revendications 1 à 8, dans laquelle ladite composition comprend en outre au moins un élément essentiel choisi dans le groupe constitué par le zinc et le sélénium.

10. Composition destinée à être utilisée selon la revendication 9, dans laquelle le zinc provient d'un sel organique, tel que le gluconate de zinc, ou d'une levure ou d'une bactérie capable d'internaliser le zinc.

11. Composition destinée à être utilisée selon la revendication 9, dans laquelle le sélénium provient d'un sel organique, tel que le gluconate de sélénium, ou d'une levure ou d'une bactérie capable d'internaliser le sélénium.

12. Utilisation d'au moins une souche bactérienne choisie dans le groupe constitué par les *Bifidobacterium adolescentis* N° DSM 18350, *Bifidobacterium adolescentis* N° DSM 18352, et *Bifidobacterium adolescentis* N° DSM 18353, et d'au moins une vitamine choisie parmi les vitamines du groupe B, pour la préparation d'une composition pharmaceutique pour le traitement de l'hyperhomocystéinémie du plasma.

13. Utilisation selon la revendication 12, dans laquelle ladite composition comprend la souche bactérienne *Bifidobacterium adolescentis* N° DSM 18352 et au moins une souche bactérienne choisie dans le groupe comprenant les *Bifidobacterium adolescentis* N° DSM 16594, *Bifidobacterium adolescentis* N° DSM 16595, *eifidobacterium breve* N° DSM 16596, *Bifidobacterium pseudocatenulatum* N° DSM 16597, *Bifidobacterium pseudocatenulatum* N° DSM 16598, *Bifidobacterium adolescentis* N° DSM 18350, *Bifidobacterium adolescentis* N° DSM 18353 et *Bifidobacterium pseudocatenulatum N°* DSM 21444.

14. Utilisation selon la revendication 13, dans laquelle ladite composition comprend lesdites *Bifidobacterium adolescentis* N° DSM 18350, *Bifidobacterium adolescentis* N° DSM 18352 et *Bifidobacterium adolescentis* N° DSM 18353.
